# EUROPEAN PATENT APPLICATION

(11) **EP 4 674 847 A1**
(43) Date of publication of application: **07.01.2026**
(21) Application number: 24917563.9
(22) Date of filing: 24.09.2024
(51) Int. Cl.: C07D 401/14, C07D 513/04, A61K 31/496, A61K 51/04, A61K 103/10, A61P 35/00

(54) **NOVEL SCAFFOLD 99MTC-FAPI DIAGNOSTIC PROBE AND USE THEREOF IN PREPARATION OF DRUG OR REAGENT FOR DIAGNOSING TUMOR**

(30) Priority: 07.05.2024 CN 202410553204
(71) Applicant: Nanjing Nuoyuan Medical Instrument Co., Ltd, Nanjing, Jiangsu 210000 (CN)
(72) Inventor: GU, Yueqing, Nanjing, Jiangsu 211198 (CN); LUO, Yang, Nanjing, Jiangsu 211198 (CN); WU, Zihan, Nanjing, Jiangsu 211198 (CN); LIN, Qiao, Nanjing, Jiangsu 211198 (CN); WANG, Feng, Nanjing, Jiangsu 211198 (CN); ZHANG, Pengjun, Nanjing, Jiangsu 211198 (CN); HAN, Zhihao, Nanjing, Jiangsu 211198 (CN)
(74) Representative: Cleanthous, Marinos
(86) International application number: PCT/CN2024/120541
(87) International publication number: WO 2025/232063

(57) **Abstract**

A bran-new skeleton 99mTc-FAPI diagnostic probe and use thereof in preparation of drugs or reagents for diagnosing tumors are provided. A fibroblast activation protein-α (FAP)-targeting dimer compound has a structure represented by the following formula. Relative to the traditional FAP inhibitor radioactive drug, a technetium-99m labeled new skeleton FAP inhibitor dimer compound has an extremely high tumor intake rate, and has a relatively high contrast between the tumor and a background and has a good in-vivo biological distribution.

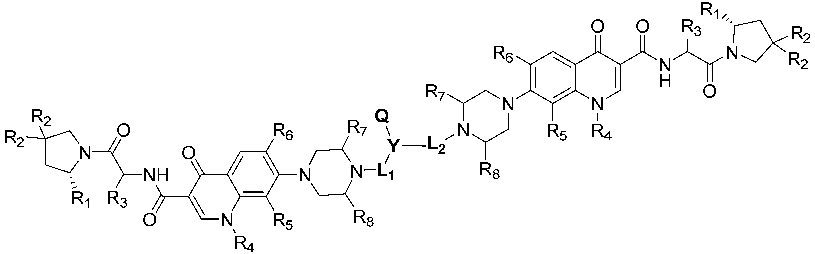

## Description

### TECHNICAL FIELD

The present disclosure relates to a bran-new skeleton 99mTc-FAPI diagnostic probe and use thereof in preparation of drugs or reagents for diagnosing tumors, belonging to the field of pharmaceutical chemistry.

### BACKGROUND

A fibroblast activation protein-α (FAP) is a transmembrane serine protease, which is mainly expressed in the surfaces of tumor-related fibroblasts, selectively highly expressed in more than 90% of epithelium-derived tumors, and highly expressed in liver cancer, colorectal cancer, pancreatic cancer, ovarian cancer, etc, but not expressed or lowly expressed in normal tissues. FAP is capable of affecting the growth of tumors through multiple mechanisms including promotion of proliferation, invasion, angiogenesis, epithelial to mesenchymal transformation, stem cell promotion, immune suppression and drug resistance. Given the widespread expression in tumors and its impact on growth of tumors through multiple mechanisms, FAP has become an important target for tumor imaging and treatment. At present, a large number of studies have shown that as a tumor broad-spectrum developer, diagnostic nuclide labeled FAPI is superior to 18F-FDG in the aspect of diagnostic performance. Most of the existing clinically developed diagnostic FAPIs are PET probes based on 18F, 68Ga and 64Cu, and there are relatively few diagnostic FAP probes based on 99mTc-SPECT.

### SUMMARY

The objective of the present disclosure: the technical problem to be solved by the present disclosure is to provide a FAP-targeting dimer compound having a relatively high tumor intake rate and developing contrast, as well as a preparation method and use thereof.

The technical solution: in order to solve the above technical problem, the present disclosure provides a FAP-targeting dimer compound or a pharmaceutically acceptable salt, hydrate and solvate thereof, and a corresponding technetium-99m radioactive nuclide marker. The compound has the following structure: 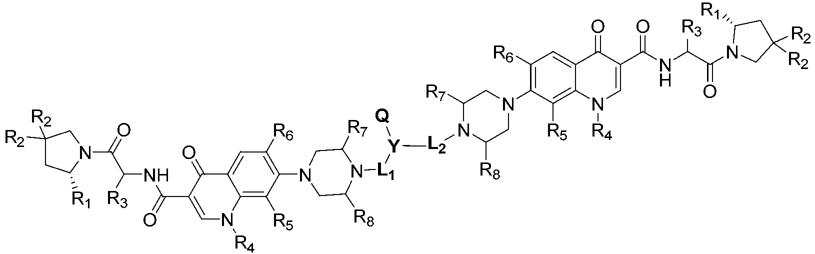
wherein, R₁ is selected from cyano, aldehyde or chloroacetyl;
R₂ is selected from hydrogen, deuterium, fluorine or chlorine;
R₃ is selected from hydrogen, deuterium, methyl, isopropyl, isobutyl, cyclopropyl or cyclobutyl;
R₄ is selected from hydrogen, methyl, ethyl, propyl, cyclopropyl or cyclobutyl;
R₅ and R₆ are independently selected from hydrogen, methyl, fluoride, chlorine, hydroxyl or methoxyl;
R₇ and R₈ are identically or differently independently selected from hydrogen, methyl, halogen or carbonyl;
As link chains Linkers, L₁ and L₂ are the same or different, are independently selected from, but not limited to, at least one of aliphatic carbon chains, polyethylene glycol chains and amino acid chains of different lengths, and comprise side chains jointed via different reactions; and the reaction comprises at least one of an amide condensation reaction, an ester condensation reaction, a substitution reaction or a click chemistry reaction;
Y is selected from any one of: 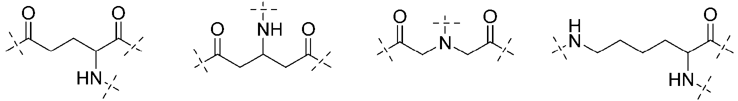 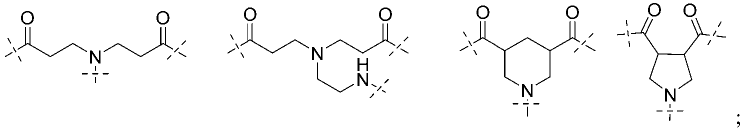
Q is a hydrogen atom, or as a nuclide chelating group moiety, is selected from any one group that can undergo chelating coordination with technetium-99mTc.

In the preferred solution of the present disclosure, as link chains Linkers, the L₁ and L₂ are independently selected from aliphatic carbon chains, polyethylene glycol chains and amino acid chains of different lengths and comprise side chains jointed via different reactions; and the reaction comprises at least one of an amide condensation reaction, an ester condensation reaction, a substitution reaction or a click chemistry reaction.

The L₁ and L₂ are independently selected from any one of the following structures: 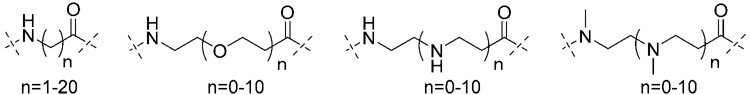 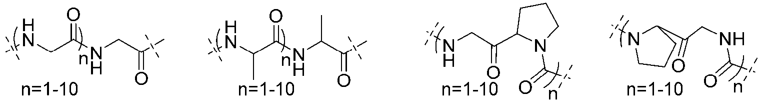 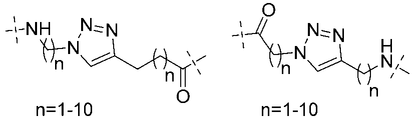 wherein, the Q is a hydrogen atom, or as a nuclide chelating group moiety, is selected from any one of: 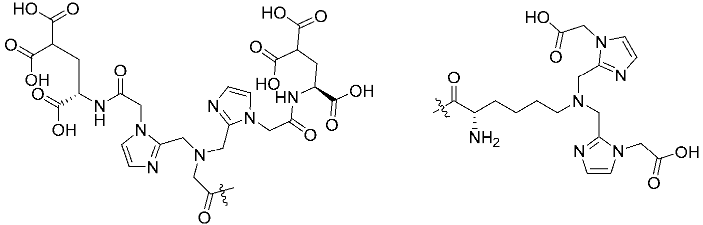 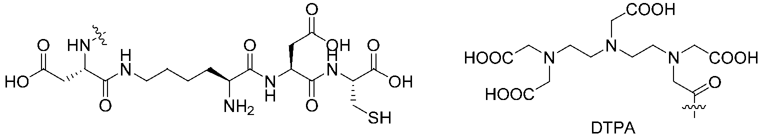 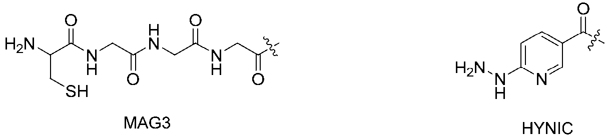 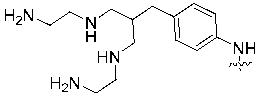 wherein, Q is preferentially selected as 6-hydrazinopyridine-3-carboxylic acid (HYNIC).

In the preferred solution of the present disclosure, the compound is selected from any one of:

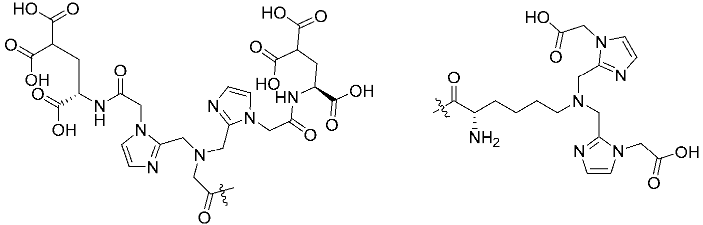

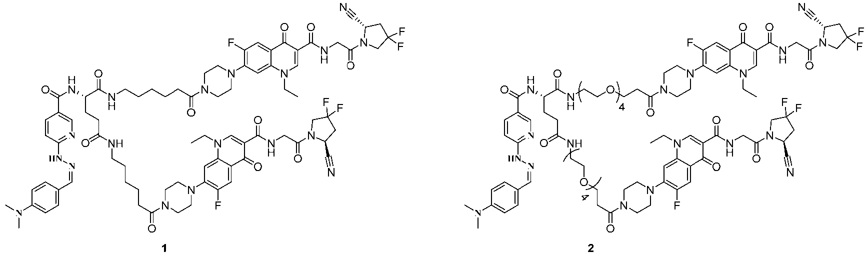

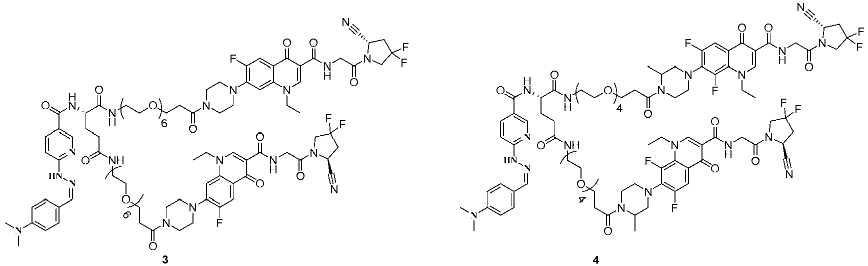

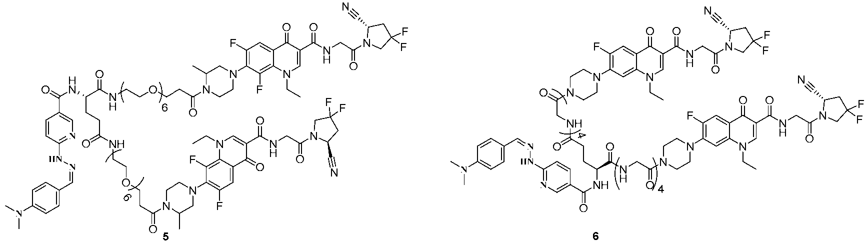

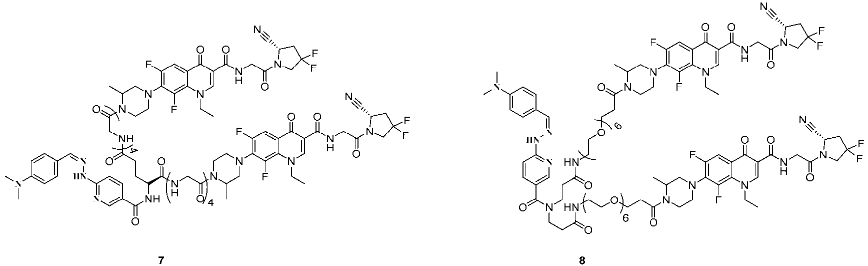

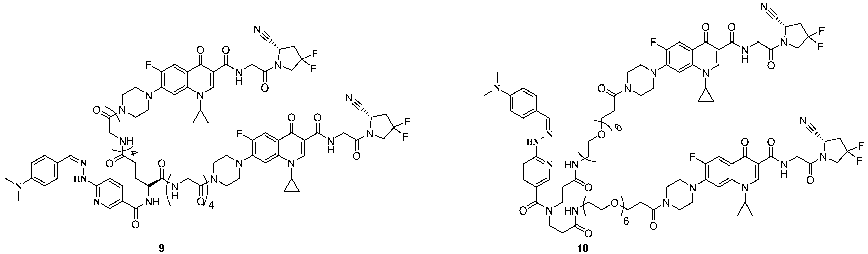

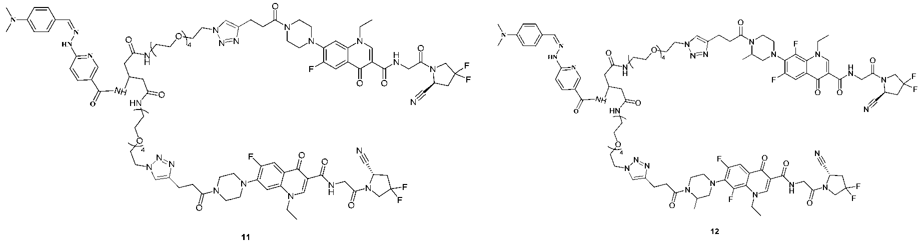

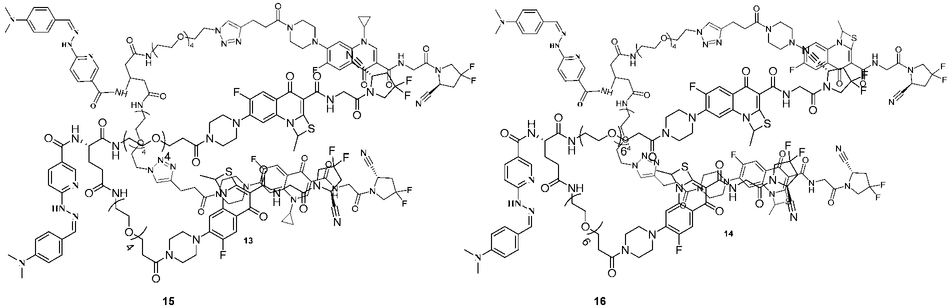

The present disclosure further provides a radioactive nuclide technetium-99m labeled FAP-targeting dimer compound, comprising a pharmaceutically acceptable salt, hydrate, solvate and tautomer of the dimer compound.

The present disclosure further provides a synthesis method and technetium-99m labeling method for preparing the dimer compound, comprising:
(1) synthesis of a targeted ligand: piperaziquinolone reacts with di-tert-butyl dicarbonate to form a tert-butyloxycarbonyl protected intermediate **I;**
   a corresponding tert-butyloxycarbonyl protected amino acid reacts with pyrrolidine having different substituents to form a dipeptide analogue, and then tert-butyloxycarbonyl is removed from the dipeptide analogue using trifluoroacetic acid to obtain an intermediate **II**;
   the intermediate **I** and the intermediate **II** undergo a condensation reaction, after the reaction is ended, a reaction solution is dropwise added into water to precipitate out a solid, the precipitated solid is filtered and dried, and a tert-butyloxycarbonyl protecting agent is then removed from the dried solid using trifluoroacetic acid to obtain a targeted ligand **III;**
(2) synthesis of a dimer: the targeted ligand reacts with a link chain Linker (L₁/L₂) with different protecting groups, and then the above reaction product undergoes a deprotection reaction to obtain an intermediate **IV;**
   the intermediate **IV** reacts with Y having a protecting agent, and then the above reaction product undergoes deprotection to obtain an intermediate **V;**
   the intermediate **V** reacts with a ligand Q which can chelate technetium-99m to obtain an FAP-targeting prodrug; and
(3) the FAP-targeting prodrug compound is labeled according to the existing wet method or dry method so as to prepare the radioactive nuclide technetium-99m labeled FAP-targeting dimer compound having a new skeleton in the present disclosure.

The present disclosure further provides a pharmaceutical composition, comprising the FAP-targeting dimer compound having the new skeleton (piperaziquinolone), the FAP-targeting dimer compound that can be labeled by radioactive nuclide technetium-99m, the radioactive nuclide technetium-99m labeled FAP-targeting dimer compound, or their pharmaceutically acceptable salts, hydrates, solvates, tautomers and racemates, and any pharmaceutically acceptable carriers and/or excipient compositions.

The present disclosure further provides use of the FAP-targeting dimer compound having the new skeleton (piperaziquinolone), the FAP-targeting dimer compound that can be labeled by radioactive nuclide technetium-99m, the radioactive nuclide technetium-99m labeled FAP-targeting dimer compound, or their pharmaceutically acceptable salts, hydrates, solvates, tautomers and racemates, and any pharmaceutically acceptable carriers and/or excipient compositions in diagnosis or treatment of diseases characterized by FAP positivity.

The diseases characterized by FAP positivity comprise tumors highly expressing FAP.

The diseases characterized by overexpression of FAP include, but are not limited to, various malignant tumors (breast cancer, lung cancer, pancreatic cancer, stomach cancer, liver cancer, colorectal cancer, thyroid cancer, etc.) and non-tumor diseases (myocardial infarction, rheumatoid arthritis, heart failure, kidney diseases, pulmonary fibrosis, tissue remodeling and scars, etc.).

The present disclosure provides a kit, comprising an FAP-targeting dimer compound that can be labeled by radioactive nuclide technetium-99m, a ligand that can form coordination with technetium-99m according to the existing method, a reducing agent, an additive, a stabilizer and an instruction for diagnosis of a disease.

The benefits: compared with the prior art, the present disclosure has the following significant advantages: 1, the present disclosure develops a class of bran-new skeletons based on piperaziquinolone, and an FAP target probe derived based on the bran-new skeleton has relatively higher tumor intake rate and developing contrast; 2, to further improve the tumor intake rate, in-vivo metabolic distribution is optimized, the physical and chemical properties of a new molecule are balanced through selection and optimization of different link chains, a dimer probe based on the skeleton is designed, the dimer probe has greatly improved tumor intake rate, quicker tumor targeting and longer retention ability compared with a monomer probe so that it is extremely low in physiological intake of non-target organs (liver, lung, kidney, pancreas, intestine, blood pool, thyroid, etc.), and has good pharmacokinetic properties and clinical application prospects.

### BRIEF DESCRIPTION OF THE DRAWINGS

FIG. 1 is a ¹H NMR spectrogram of compound a8 in Example 1 of the present disclosure;
FIG. 2 is a mass spectrogram of compound a11 in Example 1 of the present disclosure;
FIG. 3 is a mass spectrogram of compound a13 in Example 1 of the present disclosure;
FIG. 4 is a mass spectrogram of compound 1 in Example 1 of the present disclosure;
FIG. 5 is a mass spectrogram of compound c11 in Example 3 of the present disclosure;
FIG. 6 is a mass spectrogram of compound 3 in Example 3 of the present disclosure;
FIG. 7 is a ¹H NMR spectrogram of compound d8 in Example 4 of the present disclosure;
FIG. 8 is a mass spectrogram of compound f11 in Example 6 of the present disclosure;
FIG. 9 is a mass spectrogram of compound f12 in Example 6 of the present disclosure;
FIG. 10 is a mass spectrogram of compound 6 in Example 6 of the present disclosure;
FIG. 11 is a ¹H NMR spectrogram of compound i8 in Example 9 of the present disclosure;
FIG. 12 is a mass spectrogram of compound 11 in Example 11 of the present disclosure;
FIG. 13 shows a developing result of 99mTc labeled compound 5 in U87MG mice in Examples of the present disclosure;
FIG. 14 shows a developing result of 99mTc labeled compound 9 in U87MG mice in Examples of the present disclosure;
FIG. 15 is an in-vitro distribution statistic diagram of tumors and important organs after 99mTc labeled compounds 5 and 9 are injected into U87MG mice for 1 h in the present disclosure: A: 99mTc-5; B: 99mTc-9;
FIG. 16 is a scale diagram of an intake graph of tumors to important organs after 99mTc labeled compounds 5 and 9 are injected into U87MG mice for 1 h in the present disclosure: A: 99mTc-5; B: 99mTc-9;
FIG. 17 is an in-vitro distribution statistic diagram after tumor is blocked after 99mTc labeled compounds 5 and 9 are injected into U87MG mice for 1 h in the present disclosure, A: 99mTc-5; B: 99mTc-9; and
FIG. 18 is an intake scale diagram of 99mTc labeled compounds 5 and 9 in U87MG cells in the present disclosure: A: 99mTc-5; B: 99mTc-9.

### DETAILED DESCRIPTION OF THE EMBODIMENTS

Next, the technical solution of the present disclosure will be further illustrated in conjunction with drawings.

A synthesis method in examples of the present disclosure includes two parts, i.e., synthesis of a targeted ligand and synthesis of a dimer. Compounds a1, a2, a5, a9, Boc-Glu, HYNIC-NHS, b9, c9, d5, i5, 4-acetylsalicylic acid, k8, k9 and o5 are purchased from Shanghai Bide Technology Co., Ltd. f9 is purchased from Jiangsu Aikang Biological Research and Development Co., Ltd, U87MF cells are purchased from Nanjing Kebai Biotechnology Co., Ltd, naked mice (balb/c-nu) are purchased from Nanjing Annocon Biotechnology Co., Ltd, and a sodium pertechnetate solution is purchased from Nanjing Atomic High tech Pharmaceutical Co., Ltd.

### Example 1 Synthesis of compound 1

### 1. Preparation of targeted ligand compound a8

### Synthesis route:

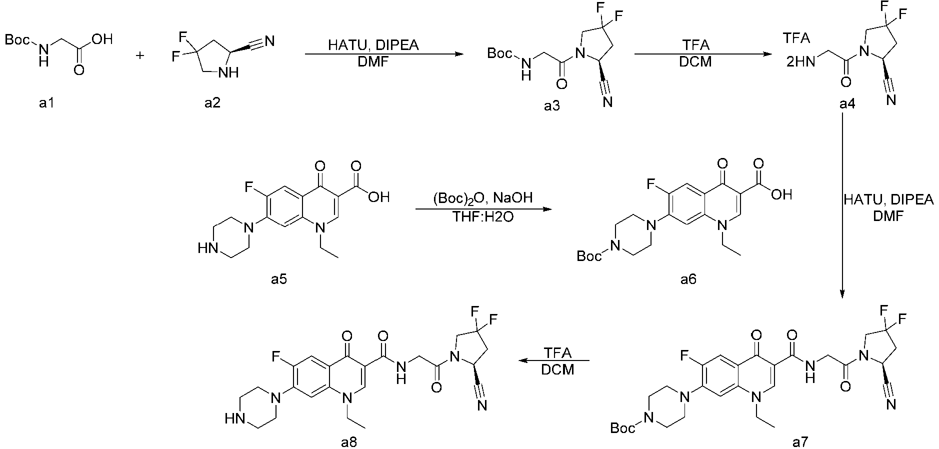

### (1) Synthesis of compound a3

N-Boc glycine a1 (175 mg, 1 mmol) and (S)-4,4-difluoropyrrolidine-2-formonitrile hydrochloride a2 (132 mg, 1 mmol) were dissolved into 5 mL of N,N-dimethylformamide (DMF), and 2-(7-azabenzotriazole)-N,N,N',N'-tetramethylurea hexafluorophosphate (HATU, 418 mg, 1.1 mmol) and N-ethyldiisopropylamine (DIPEA, 521 µL, 3 mmol) were then added into the above solution to react for 2 h at room temperature. The completion of the reaction was detected via thin layer chromatography (TLC), and then a reaction solution was concentrated. The concentrated reaction solution was dissolved with ethyl acetate, and then successively washed with water and a saturated saline solution. And an organic layer was dried and concentrated over anhydrous sodium sulfate.

### (2) Synthesis of compound a4:

The concentrated a3 was added into a mixed solution (DCM : TFA=10 : 1, v/v) to react for 1 h at room temperature. The reaction solution was concentrated. The concentrated reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound a4 (171 mg, yield 60%). **MS** (ESI): 190.20 [M+H]⁺.

### (3) Synthesis of compound a6:

1-ethyl-6-fluoro-1,4-dihydro-4-oxo-7-piperazin-3-quinoline carboxylic acid (a5, 1 g, 3.13 mmol) was dissolved into 100 mL of mixed solution (tetrahydrofuran : water=1:1, v/v), 2 M of NaOH (1.75 mL) was then added into the above mixed solution and stirred at room temperature until the solution was clarified, and then (Boc)₂O (0.75 g, 3.44 mmol) was added into the above clarified solution and stirred at room temperature overnight. After the reaction was completed, the reaction solution was subjected to vacuum concentration to remove THF, the pH of the reaction solution was adjusted to 7 with citric acid so that a white solid was precipitated out and underwent suction filtration, and subsequently the white solid after undergoing suction filtration was washed 3 times using 30 mL of water and then dried in vacuum to obtain 1.3 g of white solid with a yield of 98%.

### (4) Synthesis of compound a7:

a6 (419 mg, 1 mmol) and a4 (287 mg, 1 mmol) were dissolved into 5 mL of DMF, and HATU (418 mg, 1.1 mmol) and DIPEA (521 µL, 3 mmol) were then added into the above mixed solution to react for 2 h at room temperature. The completion of the reaction was detected via TLC, and then a reaction solution was concentrated. The concentrated reaction solution was dissolved with ethyl acetate, and then successively washed with water and a saturated saline solution. And an organic layer was dried and concentrated over anhydrous sodium sulfate. Sample loading was performed by a wet method, and the loaded sample was separated and purified via column chromatography to obtain a compound a7 (319 mg, yield 54%).

### (5) Synthesis of compound a8:

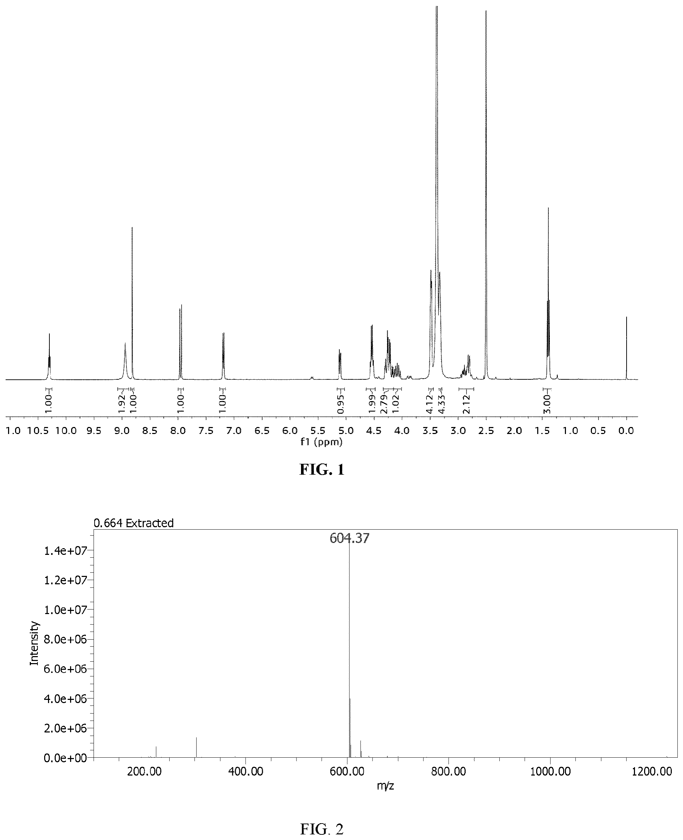

a7 (590 mg, 1 mmol) was added into a mixed solution (dichloromethane : trifluoroacetic acid=10 : 1, v/v) to react for 1 h at room temperature, the reaction solution was concentrated, the concentrated reaction solution was separated and purified via C18 reverse liquid preparative chromatography (a 10% acetonitrile aqueous solution increased to 50% in an equal proportion, 1% of acetonitrile content increased per minute, and a flow rate is 2 mL/min), and then the separated and purified product is freeze-dried to obtain a compound a8 (490 mg, yield 100%). The ¹H NMR spectrogram of the compound a8 is as shown in FIG. 1. MS (ESI): 491.35[M+H]⁺. ¹H NMR (400 MHz, DMSO-*d*₆) δ 10.30 (t, *J* = 5.2 Hz, 1H), 8.94 (s, 2H), 8.81 (s, 1H), 7.95 (d, *J* = 13.2 Hz, 1H), 7.19 (d, *J* = 7.2 Hz, 1H), 5.11 (dd, *J* = 9.0*,* 2.9 Hz, 1H), 4.54 (q, *J* = 7.0 Hz, 2H), 4.24 (qd, *J* = 17.8, 5.2 Hz, 3H), 4.09 (dt, *J* = 21.2, 10.3 Hz, 1H), 3.49 (dd, *J* = 6.7, 3.6 Hz, 4H), 3.33 (s, 4H), 2.99 - 2.73 (m, 2H), 1.40 (t, *J* = 7.1 Hz, 3H).¹³C NMR (101 MHz, DMSO-*d*₆) δ 174.50, 168.61, 164.71, 158.30, 154.10, 151.64, 148.07, 143.84, 136.92, 122.69, 118.24, 112.33, 112.11, 110.71, 106.78, 52.05, 48.90, 47.19, 44.60, 43.22, 41.86, 36.92, 14.94_{∘}

### 2. Synthesis of dimer compound 1:

### Synthesis route:

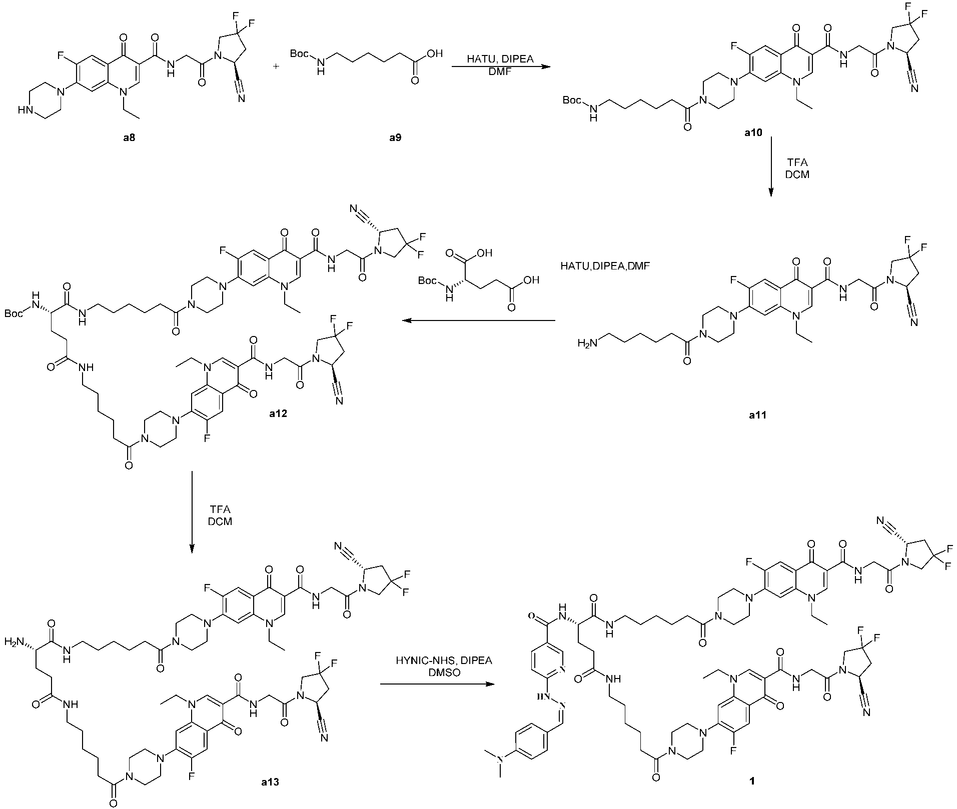

### (1) Synthesis of compound a10:

a8 (100 mg, 0.2 mmol) and a9 (47 mg, 0.2 mmol) were dissolved into 2 mL of DMF, and HATU (83 mg, 0.24 mmol) and DIPEA (104 µL, 0.6 mmol) were then added in to the above mixed solution to react for 2 h at room temperature. The completion of the reaction was detected via TLC, and then a reaction solution was concentrated. The concentrated reaction solution was dissolved with ethyl acetate, and then successively washed with water and a saturated saline solution. And an organic layer was dried and concentrated over anhydrous sodium sulfate.

### (2) Synthesis of compound a11:

The concentrated a10 was added into a mixed solution (DCM : TFA=10 : 1, v/v) to react for 1 h at room temperature. The reaction solution was concentrated. The concentrated reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound a11 (96.68 mg, yield 80%). The mass spectrogram of the compound a11 is as shown in FIG. 2. **MS:** Ms =604.37.

### (3) Synthesis of compound a12:

a11 (60.4 mg, 0.1 mmol) and Boc-Glu (12.35 mg, 0.05 mmol) were dissolved into 1 mL of DMF, and HATU (47.5 mg, 0.125 mmol) and DIPEA (52.16 µL, 0.3 mmol) were then added into the above mixed solution to react for 14 h at room temperature. Sample loading was performed by a wet method, and the loaded sample was separated and purified via column chromatography (DCM : MeOH=100 : 2) to obtain a compound a12 (86.48 mg, yield 61%).

### (4) Synthesis of compounda13:

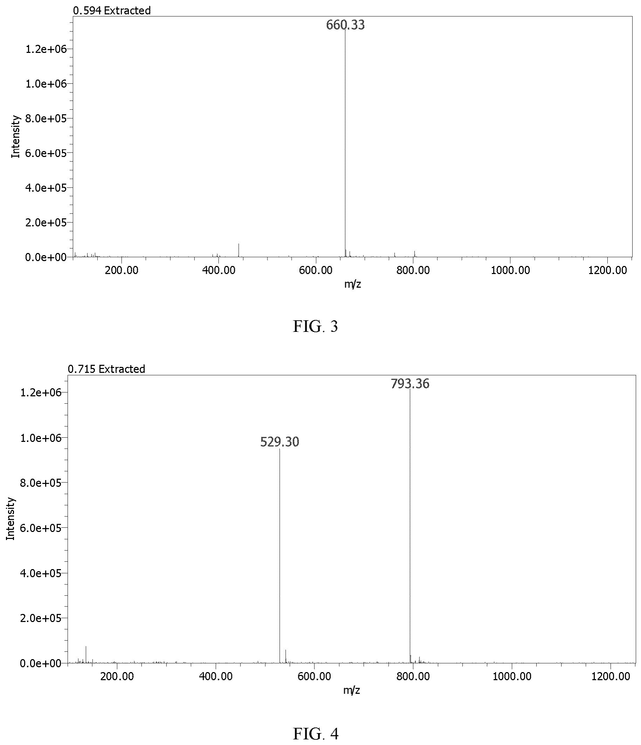

The concentrated a12 was added into a mixed solution (DCM : TFA=10 : 1, v/v) to react for 1 h at room temperature. The reaction solution was concentrated. The concentrated reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound a13 (120.53 mg, yield 85%). The mass spectrogram of the compound a13 is as shown in FIG. 3. **MS:** Ms/2 =660.3.

### (5) Synthesis of compound 1:

The freeze-dried a13 (131.7 mg, 0.1 mmol) and 6-hydrazinonicotinic acid succinimide ester hydrochloride (HYNIC-NHS, 7 mg, 0.2 mmol) were dissolved into 2 mL of dimethyl sulfoxide (DMSO), DIPEA (69.54 µL, 0.4 mmol) was dropwise added, and the above materials were stirred at room temperature overnight. The above reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound 1 (110.9 mg, yield 70%). The mass spectrogram of the compound 1 is as shown in FIG. 4. **MS:** Ms/2 =793.36.

### Example 2 Synthesis of compound 2

### Synthesis route:

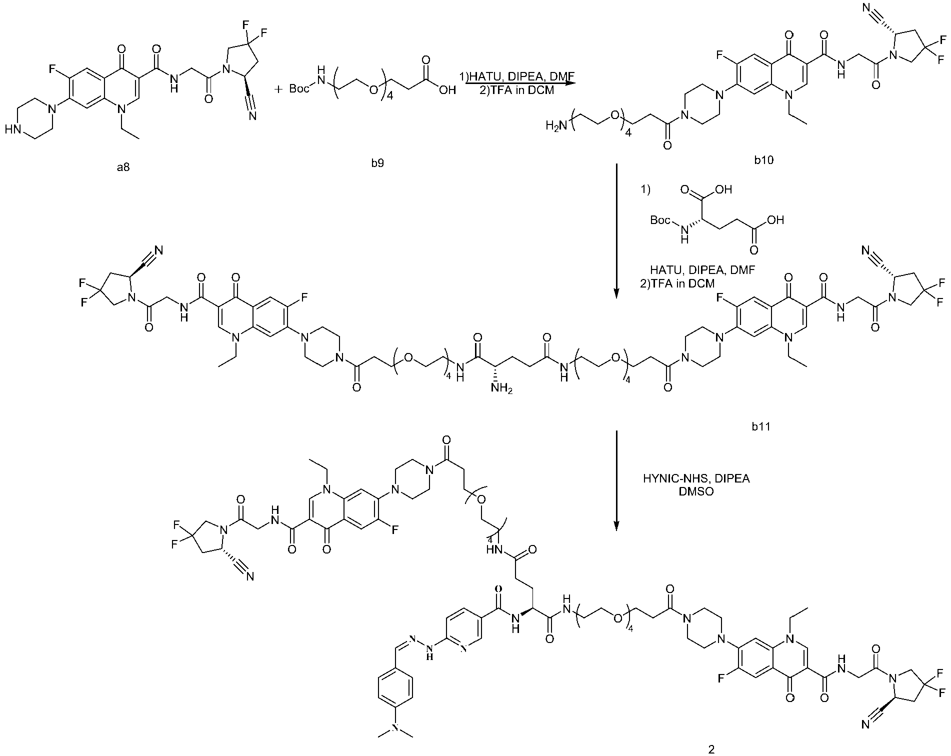

### 1. Synthesis of compound b10:

a8 (49 mg, 0.1 mmol) and 5,8,11,14-tetraoxa-2-azaheptadecanedioic acid 1-tert-butyl ester b9 (36.8 mg, 0.1 mmol) were dissolved into 1 mL of DMF, and HATU (45.60 mg, 0.12 mmol) and DIPEA (52.16 µL, 0.3 mmol) were then added into the above mixed solution to react for 2 h at room temperature. The completion of the reaction was detected via TLC, and then a reaction solution was concentrated. The concentrated reaction solution was dissolved with ethyl acetate and then successively washed with water and a saturated saline solution. And an organic layer was dried and concentrated over anhydrous sodium sulfate. The concentrated solid was added into a mixed solution (DCM : TFA=10 : 1, v/v) to react for 2 h at room temperature, the reaction solution was concentrated, the concentrated reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound b10 (44.4 mg, yield 60%).

### 2. Synthesis of compound b11:

b10 (159.1 mg, 0.1 mmol) and Boc-Glu (12.4 mg, 0.05 mmol) were dissolved into 1 mL of DMF, and HATU (47.5 mg, 0.125 mmol) and DIPEA (52.2 µL, 0.3 mmol) were then added into the above mixed solution to react at room temperature overnight. The completion of the reaction was detected via TLC, and then a reaction solution was concentrated. The concentrated reaction solution was dissolved with ethyl acetate and then successively washed with water and a saturated saline solution. And an organic layer was dried and concentrated over anhydrous sodium sulfate. Sample loading was performed by a dry method and the loaded sample was separated and purified via column chromatography (DCM : MeOH=100 : 2) to obtain a white solid, then the white solid was added into the mixed solution (DCM : TFA=10: 1, v/v) to react for 1 h at room temperature, and the reaction solution was then concentrated. The concentrated reaction solution was separated and purified via C18 reverse liquid preparative chromatography, and then the separated and purified product was freeze-dried to obtain a compound b11 (95.5 mg, yield 61%). **MS:** Ms/2 =794.33.

### 3. Synthesis of compound 2:

The freeze-dried b11 (159.1 mg, 0.1 mmol) and HYNIC-NHS (76 mg, 0.2 mmol) were dissolved into 2 mL of DMSO, DIPEA (69.54 µL, 0.4 mmol) was dropwise added, and the above materials were stirred at room temperature overnight. The above reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound 2 (157.8 mg, yield 75%). **MS:** Ms/2 =927.91.

### Example 3 Synthesis of compound 3

### Synthesis route:

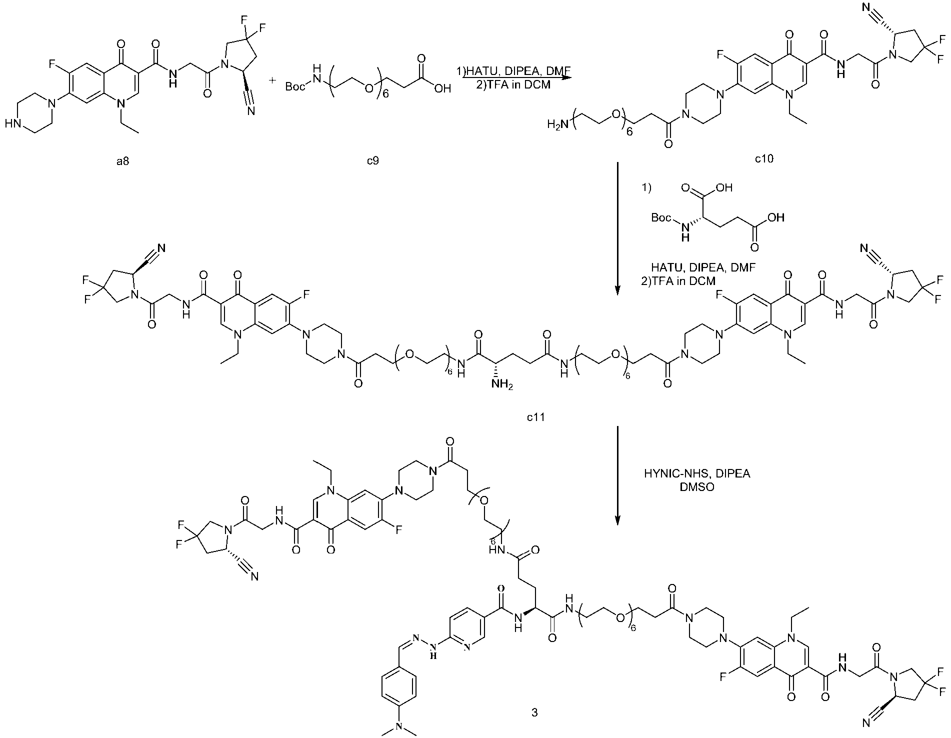

### 1. Synthesis of compound c10:

a8 (49 mg, 0.1 mmol) and c9 (45.8 mg, 0.1 mmol) were dissolved into 1 mL of DMF, and HATU (45.60 mg, 0.12 mmol) and DIPEA (52.16 µL, 0.3 mmol) were then added into the above mixed solution to react for 3 h at room temperature. The completion of the reaction was detected via TLC, and then a reaction solution was concentrated. The concentrated reaction solution was dissolved with ethyl acetate and then successively washed with water and a saturated saline solution. And an organic layer was dried and concentrated over anhydrous sodium sulfate. The concentrated solid was added into a mixed solution to react for 2 h at room temperature. The completion of the reaction was detected via TLC. The reaction solution was concentrated. The concentrated reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound c10 (48.97 mg, yield 59%).

### 2. Synthesis of compound c11:

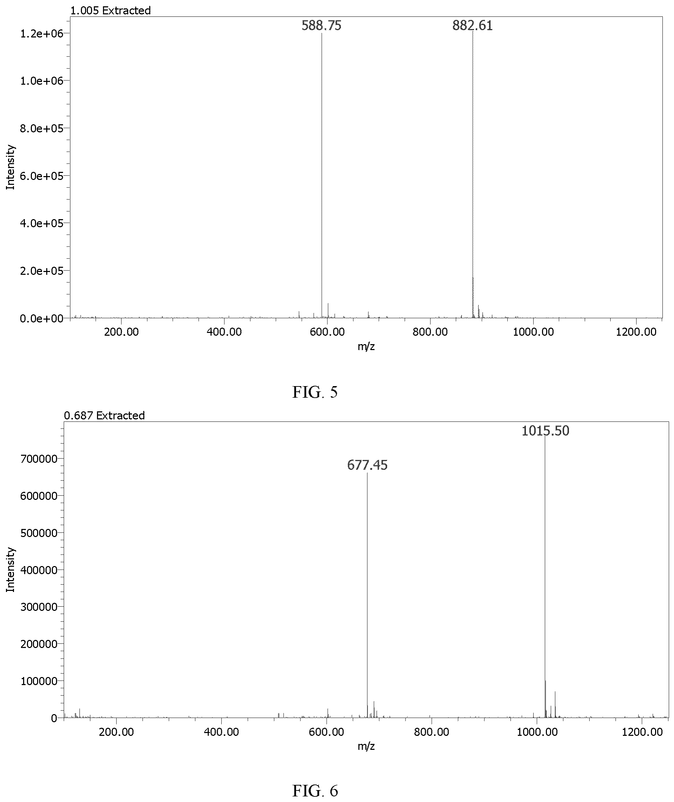

c10 (187.1 mg, 0.1 mmol) and Boc-Glu (12.4 mg, 0.05 mmol) were dissolved into 1 mL of DMF, and HATU (47.5 mg, 0.125 mmol) and DIPEA (52.2 µL, 0.3 mmol) were then added into the above mixed solution to react at room temperature overnight. The completion of the reaction was detected via TLC, and then a reaction solution was concentrated. The concentrated reaction solution was dissolved with ethyl acetate and then successively washed with water and a saturated saline solution. And an organic layer was dried and concentrated over anhydrous sodium sulfate. Sample loading was performed by a dry method and the loaded sample was separated and purified via column chromatography (DCM : MeOH=100 : 2) to obtain a white solid, then the white solid was added into the mixed solution (DCM : TFA=10 : 1, v/v) to react for 1.5 h at room temperature, The completion of the reaction was detected via TLC. The reaction solution was then concentrated. The concentrated reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound c11 (112.3 mg, yield 60%). The mass spectrogram of the compound c11 is as shown in FIG. 5. **MS:** Ms/2 =882.61.

### 3. Synthesis of compound 3:

The freeze-dried c11 (187.1 mg, 0.1 mmol) and HYNIC-NHS (76 mg, 0.2 mmol) were dissolved into 2 mL of DMSO, DIPEA (69.54 µL, 0.4 mmol) was dropwise added, and the above materials were stirred at room temperature overnight. The above reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound 3 (158.4 mg, yield 78%). The mass spectrogram of the compound 3 is as shown in FIG. 6. **MS:** Ms/2 = 1015.50.

### Example 4 Synthesis of compound 4

### Synthesis route:

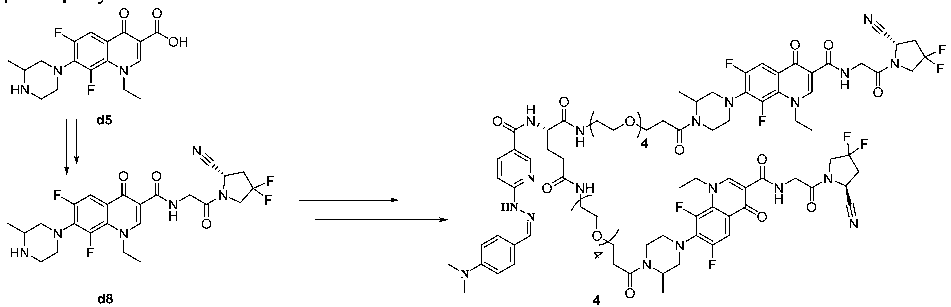

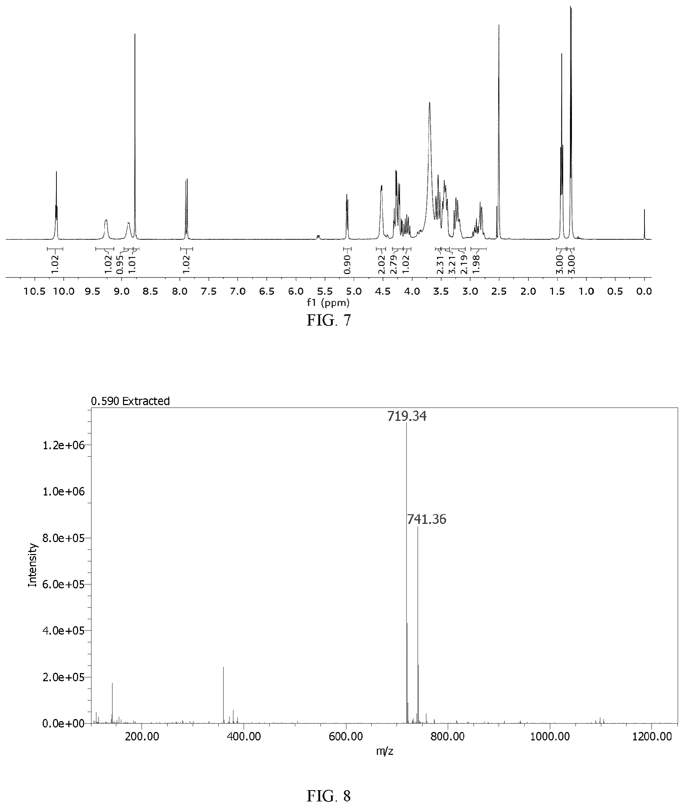

1. The synthesis of compound a8 refers to the synthesis route of a8 in Example 1. a5 was replaced with compound d5 in an equal molar ratio, with the same other materials and ratios, so as to obtain d8. The ¹H NMRspectrogram of the compound d8 is as shown in FIG. 7. ¹H NMR (400 MHz, DMSO-d6) δ 10.13 (t, J = 5.1 Hz, 1H), 9.27 (d, J = 8.0 Hz, 1H), 8.97 - 8.81 (m, 1H), 8.77 (s, 1H), 7.89 (d, J = 11.0 Hz, 1H), 5.12 (dd, J = 9.1, 2.8 Hz, 1H), 4.62 - 4.45 (m, 2H), 4.25 (qd, J = 17.8, 5.2 Hz, 3H), 4.09 (dt, J = 21.1, 10.2 Hz, 1H), 3.60 - 3.51 (m, 2H), 3.50 - 3.36 (m, 3H), 3.32 - 3.08 (m, 2H), 2.99 - 2.72 (m, 2H), 1.43 (t, J = 6.8 Hz, 3H), 1.27 (d, J = 6.5 Hz, 3H)_{∘}
2. Synthesis of compound 4: the synthesis of compound 4 refers to the synthesis route in Example 2, wherein a8 was replaced with compound d8 in an equal to molar ratio, with the same other raw materials and ratios. (Compound 4, **MS:** Ms/2 =959.42)

### Example 5 Synthesis of compound 5

The synthesis of compound 5 refers to the synthesis route in Example 3, wherein a8 was replaced with compound d8 in an equal molar ratio, with the same other raw materials and ratios, so as to obtain compound 5. (Compound 5, **MS:** Ms/2 =1047.47)

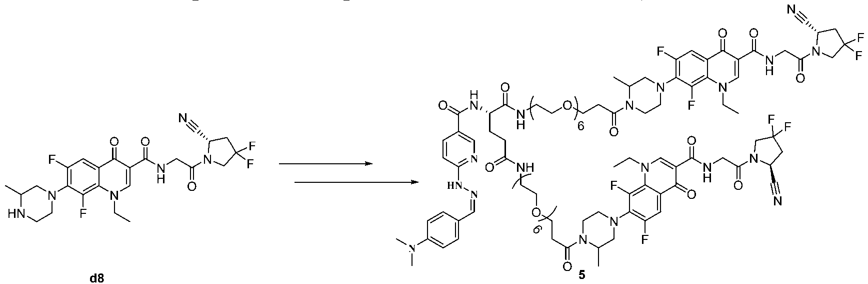

### Example 6 Synthesis of compound 6

### Synthesis route:

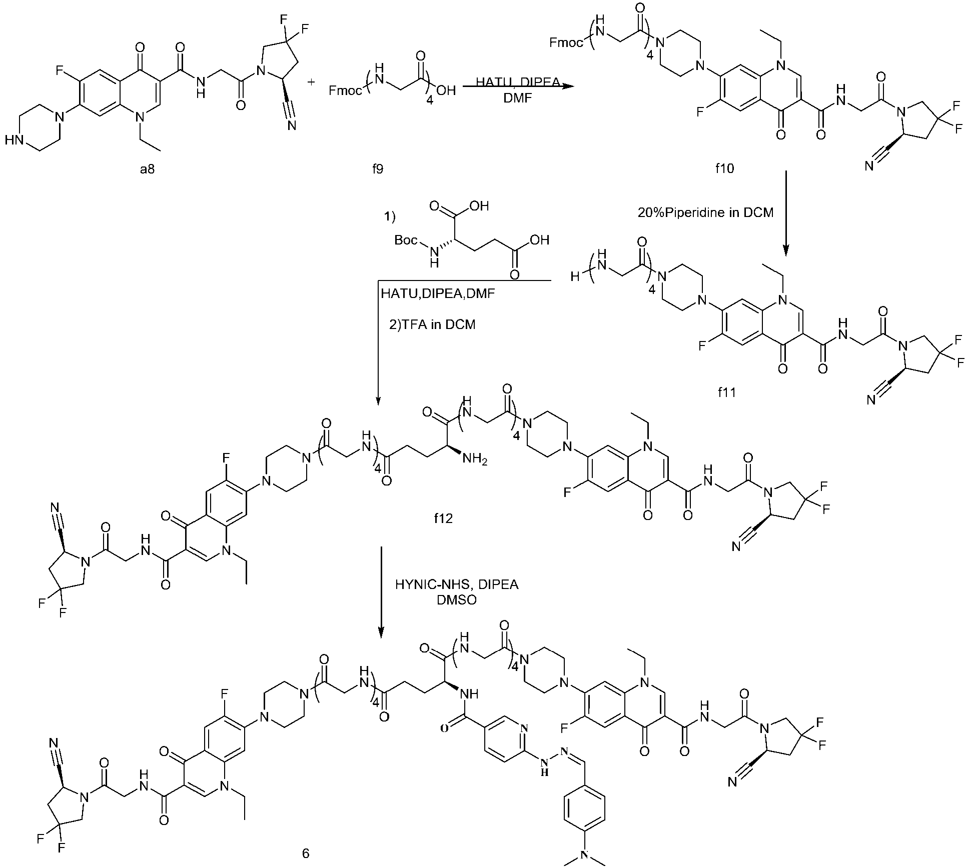

### 1. Synthesis of compound f10:

a8 (49 mg, 0.1 mmol) and f9 (51.48 mg, 0.11 mmol) were dissolved into 1 mL of DMF, and HATU (49.4 mg, 0.13 mmol) and DIPEA (52.16 µL, 0.3 mmol) were then added into the above mixed solution to react for 6 h at room temperature. The completion of the reaction was detected via TLC. The reaction solution was dropwise added into 20 mL of ice water to precipitate out a solid, the precipitated solid underwent suction filtration and then was dried at a reduced pressure to obtain a compound f10 (65. 8 mg, yield 70%).

### 2. Synthesis of compound f11:

f10 obtained by drying at a reduced pressure was added into 2 mL of mixed solution (piperidine : DCM=1 : 5, v/v) to react for 3 h at room temperature. The completion of the reaction was detected via TLC. The reaction solution was concentrated to remove piperidine. Sample loading was performed by using a dry method, and then the loaded sample was separated and purified via column chromatography (DCM : MeOH=100 : 2) to obtain a compound f11 (42.69 mg, yield 85%). The mass spectrogram of the compound f11 is as shown in FIG. 8. **MS:** Ms =719.34.

### 3. Synthesis of compound f12:

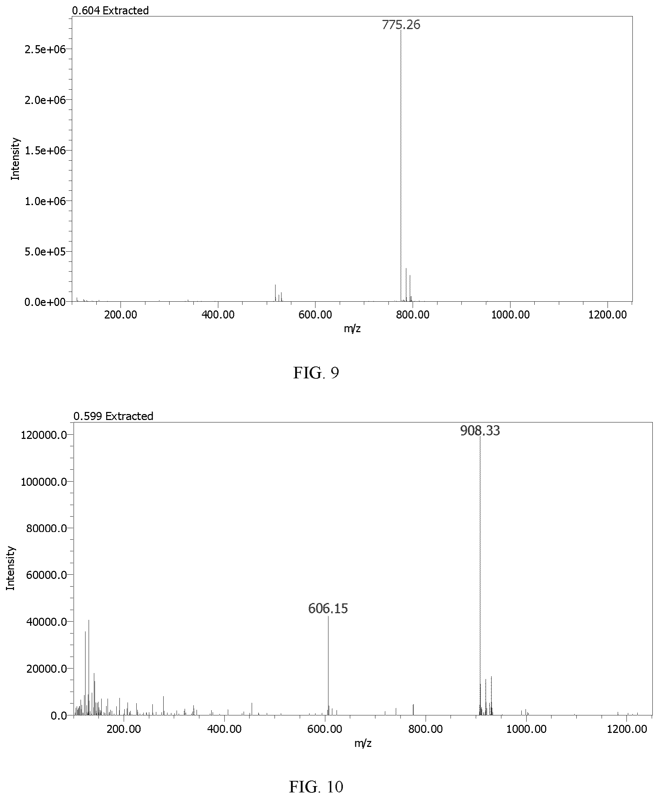

f11 (71.7 mg, 0.1 mmol) and Boc-Glu (12.4 mg, 0.05 mmol) were dissolved into 1 mL of DMF, and HATU (47.5 mg, 0.125 mmol) and DIPEA (52.2 µL, 0.3 mmol) were then added into the mixed solution to react for 14 h at room temperature. Sample loading was performed by a wet method, and the loaded sample was separated and purified via column chromatography to obtain a white solid. The white solid was added into a mixed solution (DCM : TFA=10 : 1, v/v) to react for 1 h at room temperature. The reaction solution was concentrated. The concentrated reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound f12 (94.25 mg, yield 61%). The mass spectrogram of the compound f12 is as shown in FIG. 9. **MS:** Ms/2 =775.26

### 4. Synthesis of compound 6:

The freeze-dried f12 (154.5 mg, 0.1 mmol) and HYNIC-NHS (76 mg, 0.2 mmol) were dissolved into 2 mL of DMSO, and then DIPEA (69.5 µL, 0.4 mmol) was dropwise added into the above mixed solution. The obtained solution was stirred at room temperature overnight, and then separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound 6 (108.2 mg, yield 70%). The mass spectrogram of the compound 6 is as shown in FIG. 10. **MS:** Ms/2 =908.33.

### Example 7 Synthesis of compound 7

The synthesis of compound 7 refers to the synthesis route in Example 6, wherein a8 was replaced with compound d8 in an equal molar ratio, with the same other raw materials and ratios, so as to obtain the compound 7. (Compound 7, **MS:** Ms/2 =940.84)

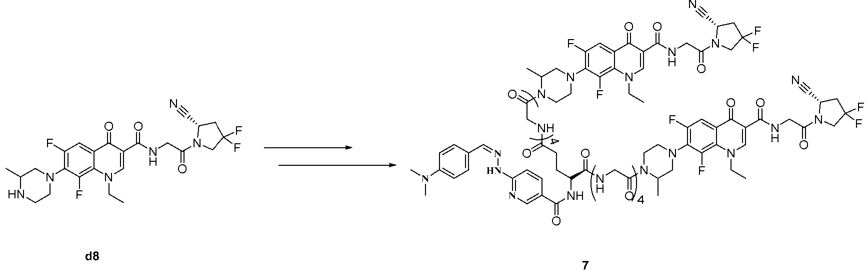

### Example 8 Synthesis of compound 8

The synthesis of compound 8 refers to the synthesis route in Example 3, wherein a8 was replaced with compound d8 in an equal to molar ratio, with the same other raw materials and ratios, so as to obtain the compound 8. (Compound 8, **MS:** Ms/2 =1054.48)

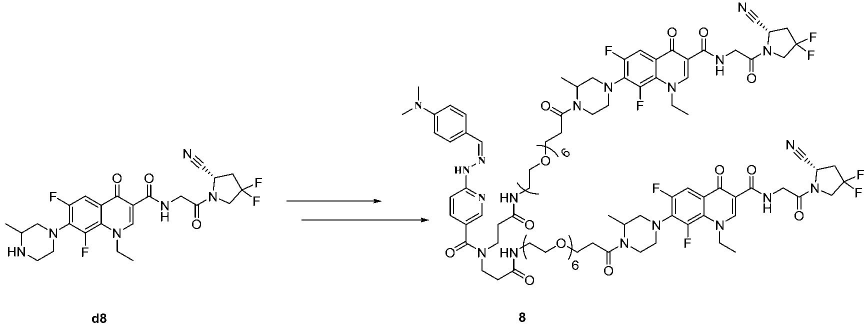

### Example 9 Synthesis of compound 9

### Synthesis route:

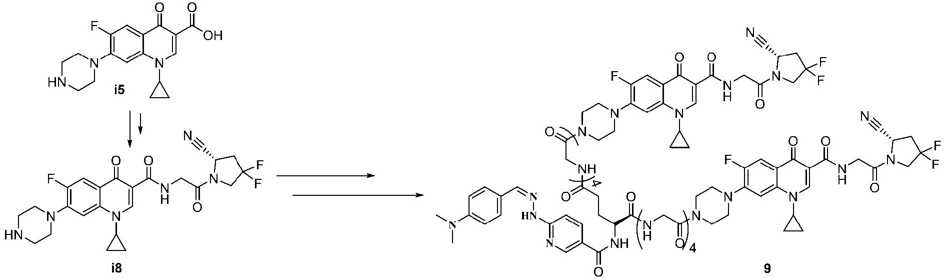

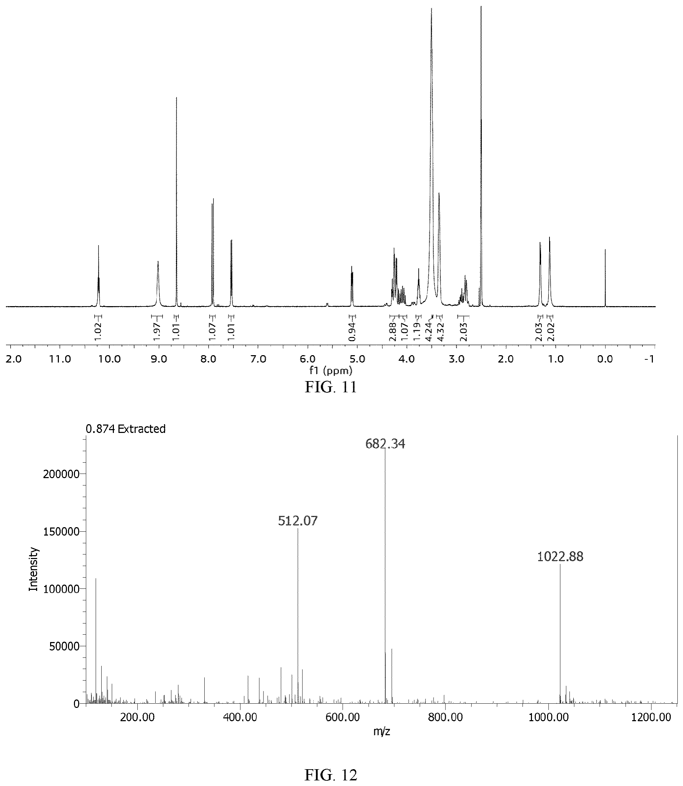

The synthesis of compound i8 refers to the synthesis route of a8 in Examples, wherein a5 was replaced with compound i5 in an equal to molar ratio, with the same other raw materials and ratios, so as to obtain the compound i8. The ¹H NMRspectrogram of the compound i8 is as shown in FIG. 11. ¹H NMR (400 MHz, DMSO-*d*₆) δ 10.22 (t, *J* = 5.2 Hz, 1H), 9.02 (s, 2H), 8.65 (s, 1H), 7.92 (d, *J* = 13.2 Hz, 1H), 7.54 (d, *J =* 7.4 Hz, 1H), 5.11 (dd, *J* = 9.1, 2.8 Hz, 1H), 4.24 (qd, *J* = 17.8, 5.2 Hz, 3H), 4.08 (dt, *J* = 21.2, 10.2 Hz, 1H), 3.77 (tt, *J* = 7.2, 4.1 Hz, 1H), 3.49 (d, *J* = 6.4 Hz, 4H), 3.35 (s, 4H), 2.98 - 2.74 (m, 2H), 1.31 (d, *J* = 6.5 Hz, 2H), 1.12 (d, *J* = 3.6 Hz, 2H)_{∘}

The synthesis of compound 9 refers to the synthesis route in Example 6, wherein a8 was replaced with the compound i8 in an equal molar ratio, with the same other materials and ratios, so as to obtain 9. (Compound 9, **MS:** Ms/2 =920.83)

### Example 10 Synthesis of compound 10

The synthesis of compound 10 refers to the synthesis route in Example 3, wherein a8 was replaced with the compound i8 in an equal molar ratio, with the same other materials and ratios, so as to obtain 10. (Compound 10, **MS:** Ms/2 =1052.47)

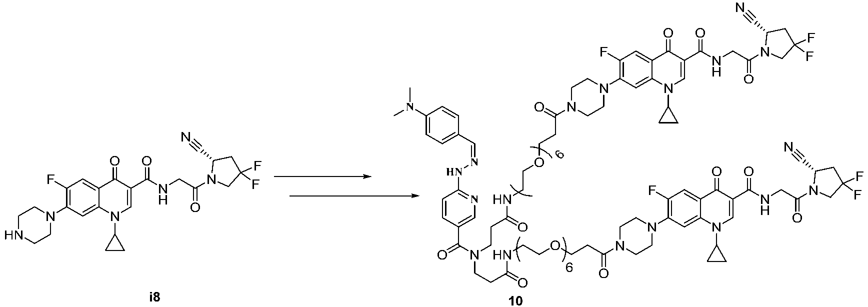

### Example 11 Synthesis of compound 11

### Synthesis route:

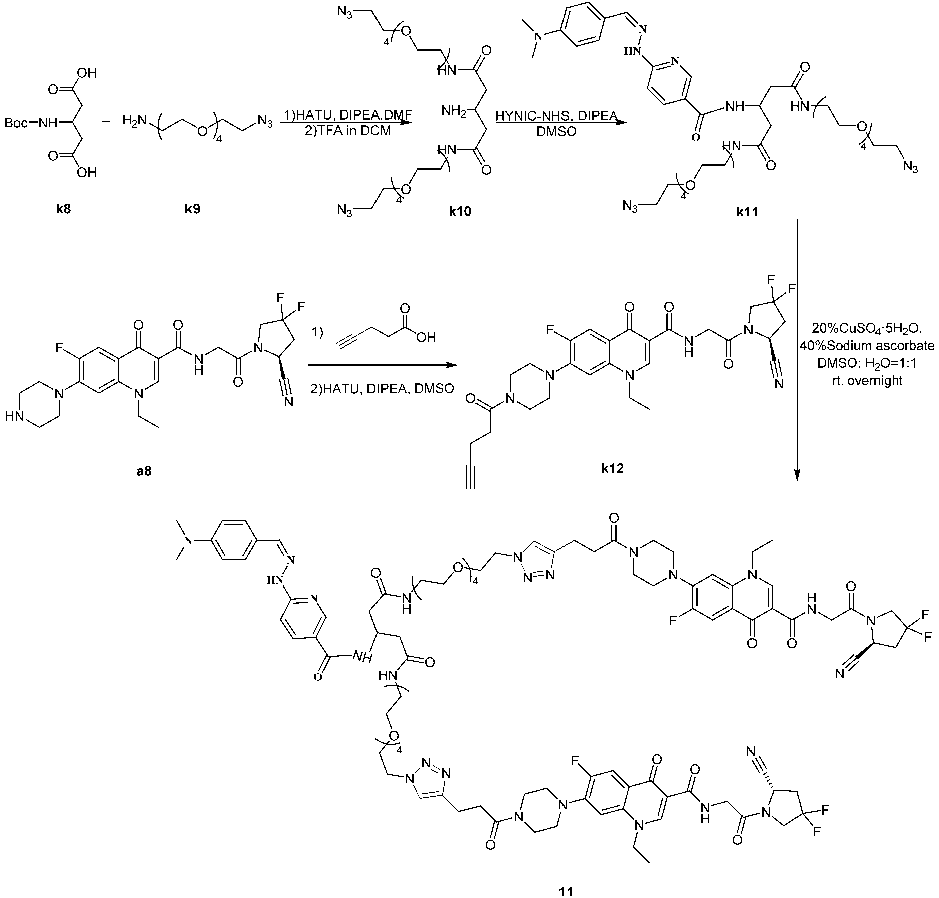

### 1. Synthesis of compound k10:

k8 (24.7 mg, 0.1 mmol) and k9 (52.4 mg, 0.2 mmol) were dissolved into 1 mL of DMF, and HATU (45.60 mg, 0.12 mmol) and DIPEA (52.16 µL, 0.3 mmol) were then added into the above mixed solution to react for 3 h at room temperature. The above reaction solution was separated and purified via C18 reverse liquid preparative chromatography, and then the separated and purified product was freeze-dried to obtain a solid compound. The freeze-dried solid compound was added into a mixed solution (DCM : TFA=10 : 1, v/v) to react for 1 h at room temperature. The reaction solution was concentrated and then separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain a compound k10 (40.6 mg, yield 64%).

### 2. Synthesis of compound k11:

The freeze-dried k10 (63.5 mg, 0.1 mmol) and HYNIC-NHS (7 mg, 0.2 mmol) were dissolved into 2 mL of DMSO, DIPEA (69.54 µL, 0.4 mmol) was dropwise added, and then the above materials were stirred at room temperature overnight. The above reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain the compound k11 (63.1 mg, yield 70%).

### 3. Synthesis of compound k12:

a8 (49.0 mg, 0.1 mmol) and 4-pentynoic acid (9.8 mg, 0.1 mmol) were dissolved into 1 mL of DMF, and HATU (41.8 mg, 0.11 mmol) and DIPEA (52.1 µL, 0.3 mmol) were then added into the above mixed solution to react for 2 h at room temperature. The completion of the reaction was detected via TLC. The reaction solution was separated and purified via C18 reverse liquid preparative chromatography (10% acetonitrile aqueous solution increased to 50% in an equal proportion, 1% of acetonitrile content increased per minute, and a flow rate was 2 mL/min).

### 4. Synthesis of compound 11:

A Cu⁺ aqueous solution was prepared by using CuSO₄·5H₂O and vitamin C sodium. k11 (90.2 mg, 0.1 mmol) and k12 (114 mg, 0.2 mmol) were dissolved into 1.5 mL of mixed solution (DMSO : H₂O=1:1, v/v), a catalytic amount of Cu⁺ aqueous solution was added into the above mixed solution, and the above materials reacted for 4 h at room temperature. The completion of the reaction was detected via TLC. The reaction solution was separated and purified via C18 reverse liquid preparative chromatography (10% acetonitrile aqueous solution increased to 50% in an equal proportion, 1% of acetonitrile content increased per minute, and a flow rate was 2 mL/min), and then the separated and purified product was freeze-dried to obtain the compound 11. The mass spectrogram of the compound 11 is as shown in FIG. 12. **MS:** Ms/2 =1022.88.

### Example 12 Synthesis of compound 12

The synthesis of compound 12 refers to the synthesis route in Example 11, wherein a8 was replaced with the compound d8 in an equal molar ratio, with the same other materials and ratios, so as to obtain 12. (Compound 12, **MS:** Ms/2 =1054.39)

### The structural formula of compound 12:

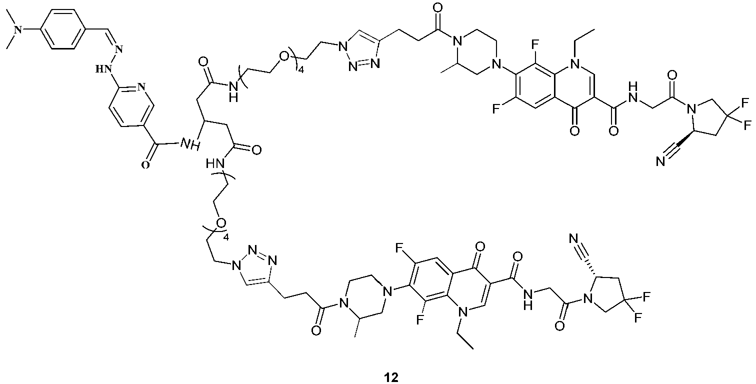

### Example 13 Synthesis of compound 13

The synthesis of the compound 13 refers to the synthesis route in Example 11, wherein a8 was replaced with a compound i8 in an equal molar ratio, with the same other materials and ratios, so as to obtain 13. (Compound 13, **MS:** Ms/2 =1034.88)

### The structural formula of the compound 13:

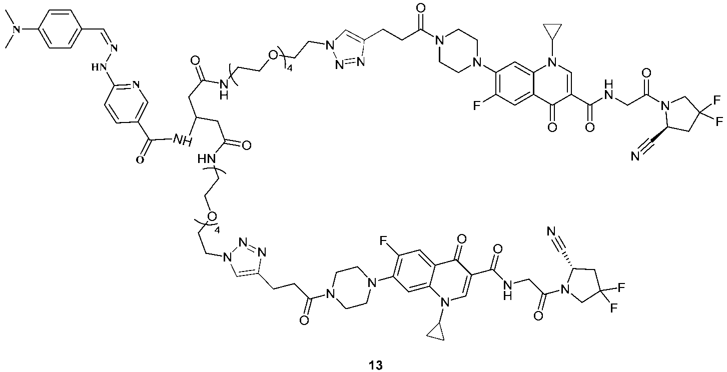

### Example 14 Synthesis of compound 14

1. The synthesis of the compound o8 refers to the synthesis route of a8 in Examples, wherein a5 was replaced with a compound o5 in an equal molar ratio, with the same other materials and ratios, so as to obtain o8.
2. The synthesis of compound 14 refers to the synthesis route in Example 11, wherein a8 was replaced with the compound o8 in an equal molar ratio, with the same other materials and ratios, so as to obtain 14. (Compound 14, **MS:** Ms/2 =1052.33)

### The structural formula of the compound 14:

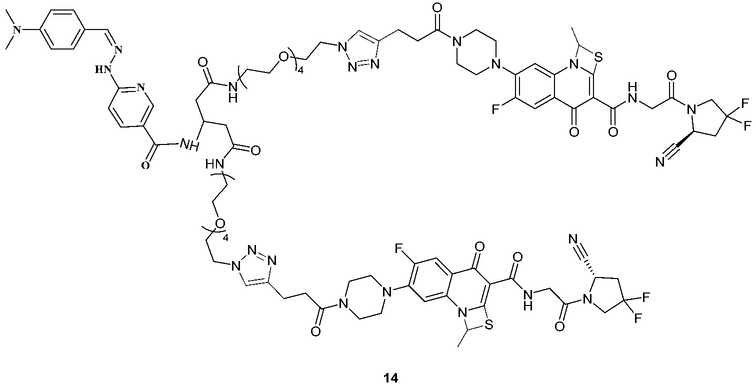

### Example 15 Synthesis of compound 15

### Synthesis route:

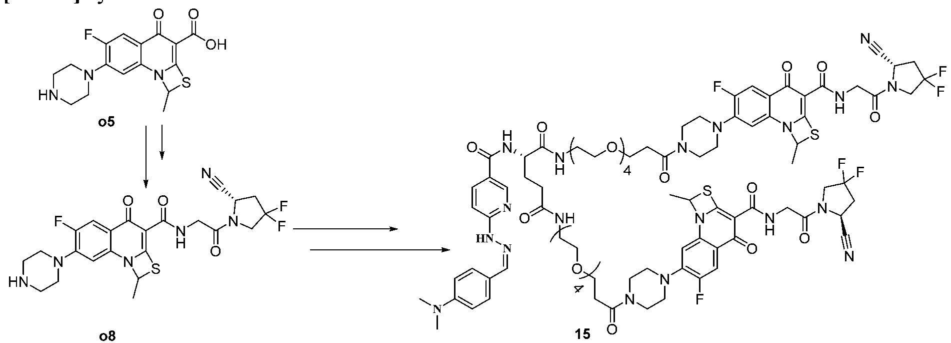

1. The synthesis of compound o8 refers to the synthesis route of a8 in Examples, wherein a5 was replaced with a compound o5 in an equal molar ratio, with the same other materials and ratios, so as to obtain o8.
2. The synthesis of compound 15 refers to the synthesis route in Example 2, wherein a8 was replaced with the compound o8 in an equal molar ratio, with the same other materials and ratios, so as to obtain 15. (Compound 15, **MS:** Ms/2 =957.37)

### Example 16 Synthesis of compound 16

### Synthesis route:

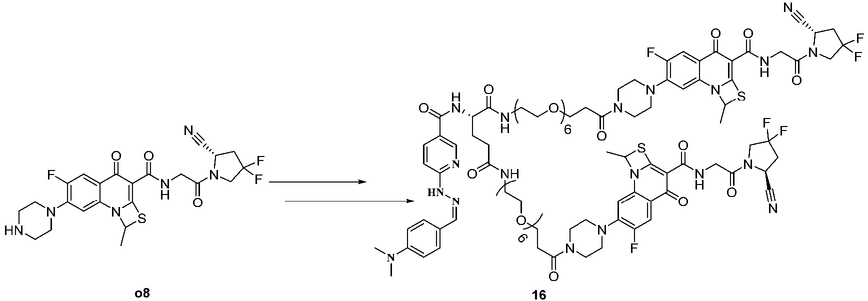

The synthesis of compound 16 refers to the synthesis route in Example 3, wherein a8 was replaced with the compound o8 in an equal molar ratio, with the same other materials and ratios, so as to obtain 16. (Compound 16, **MS:** Ms/2 =1045.42)

### Example 17 Preparation of radioactive 99mmTc labeled ligand

10 µL of sodium triphenylphosphine trisulphonate aqueous solution (150 mg/mL), 10 µL of tris(hydroxymethyl) methylglycine aqueous solution (200 mg/mL) and 1 µL of solution (1 mg/mL) of labeled precursor compound 5 or compound 9 in DMSO were added into a vial, then 2 mCi of sodium pertechnetate leacheate was added into the vial, the vial was put into metal bath and heated to react for 20 min at 100°C, and subsequently the reaction product was cooled to room temperature. C18 Cartridge was leached and activated with 10 mL of absolute ethyl alcohol and 10 mL of normal saline. The reaction solution was diluted with 2 mL of normal saline, filtered via C18 Cartridge, washed with 5 mL of normal saline, leached with 75% ethanol, diluted with normal saline and then filtered via a sterile filter membrane so as to respectively obtain an injection 99mTc-5 or 99mTc-9 of a 99mTc labeled complex.

### Example 18 Cell intake experiment

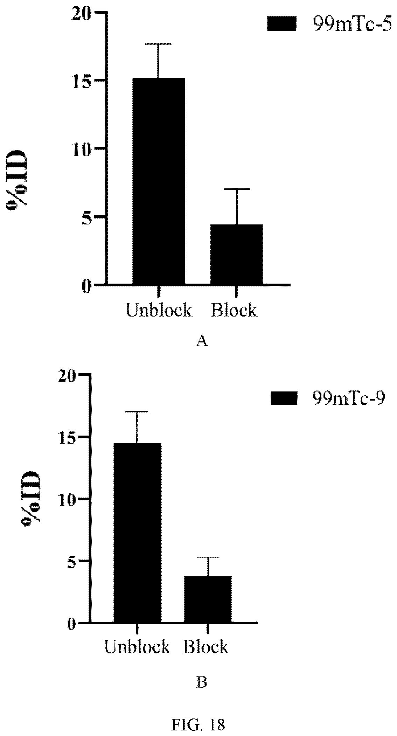

U87MG cells were inoculated into a 6-well plate (1×10⁵ cells in each well). 17.5 kBq of 99mTc-5 or 99mTc-9 was respectively added into each well. Post to the incubation for 1 h at 4°C, the cells were washed 3 times with cold PBS and collected. The related radiation quantities of the cells were measured using a γ counter. The results showed a percentage of a total addition dose per 10⁵ cells In a blocking experiment, U87MG cells and 100 fold excess of FAP inhibitor (UAMC1110) were incubated for 1 h, and then a probe 99mTc-5 or 99mTc-9 was respectively added for incubation. As shown in FIG. 18, it showed the higher tumor cell intake rate (about 15% ID) of the 99mTc-5 or 99mTc-9 probe.

### Example 19 Animal imaging experiment

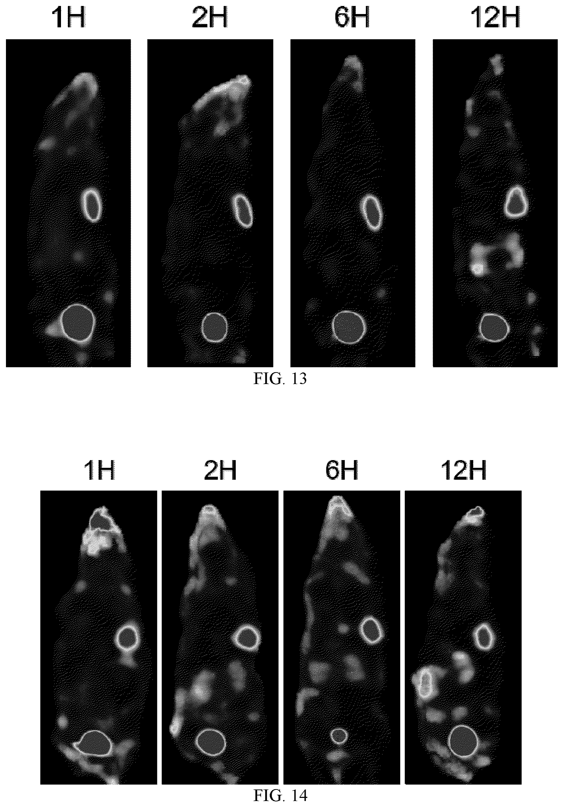

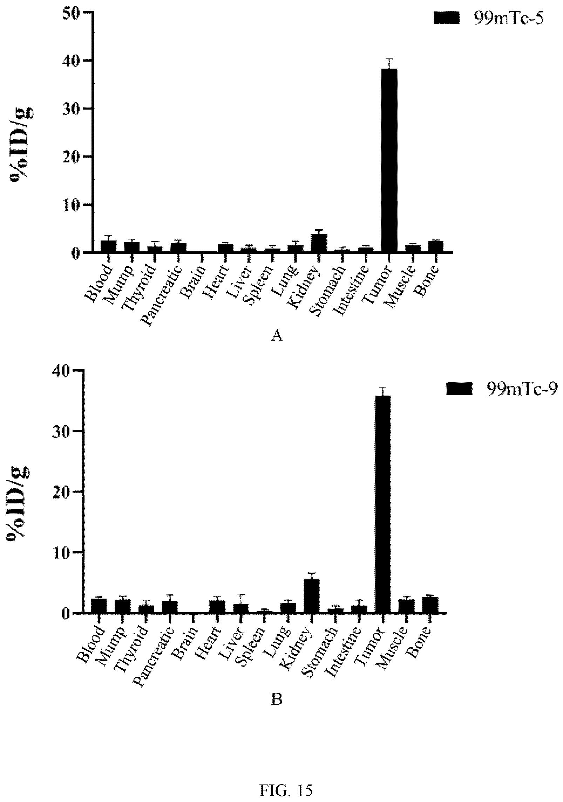

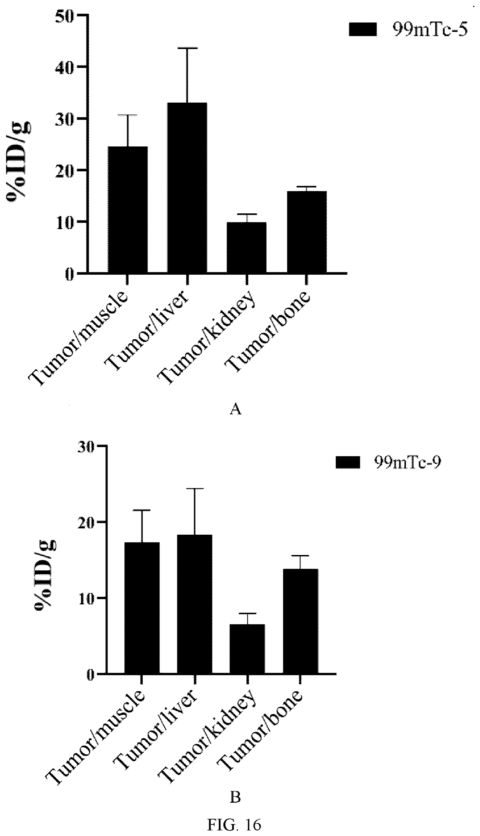

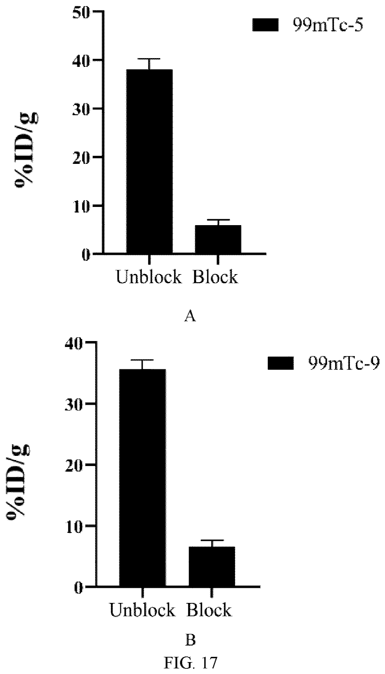

The probe 99mTc-5 or 99mTc-9 was injected into U87MG tumor-bearing mice via tail veins, with 300 µCi of technetium-labeled probe injected in each mouse. The injected tumor-bearing mice were scanned using Micro-SPECT/CT, the tumor-bearing mice were maintained in an anesthetic state through persistent inhalation of 2% isoflurane, and then imaging was performed at a time point of 1 h, 2 h, 6 h and 12 h after injection. As shown in FIG. 13 and FIG. 14, the 99mTc-5/99mTc-9 probe showed excellent tumor targeting and long retention abilities. The biological distribution of 99mTc-5 or 99mTc-9 in main organs and tumor tissues of tumor-bearing mice was further evaluated. 1.85 MBq of probe 99mTc-5 or 99mTc-9 was injected into U87MG tumor-bearing mice. After injection for 1 h, the tumor-bearing mice were isolated and weighed, and then the radiation quantity in each organ was measured using the γ counter. As shown in FIG. 15, the tumor intake rate of the probe 9mTc-5 within 1 h was up to 38.1% ID/g, and the tumor intake rate of the probe 9mTc-9 within 1 h was up to 35.7% ID/g. As shown in FIG. 16, such the probe has an excellent developing contrast. In the blocking experiment, the probe 99mTc-5 or 99mTc-9 and 100 fold excess of FAP inhibitor (UAMC1110) were co-injected into U87MG tumor-bearing mice for ex vitro distribution. The tumor intake change results were counted. As shown in FIG. 17, the 99mTc-5/99mTc-9 probe showed the specificity for targeting FAP.

## Claims

1. A fibroblast activation protein-α (FAP)-targeting dimer compound or a pharmaceutically acceptable salt thereof, wherein the FAP-targeting dimer compound has a structure represented by the following formula: 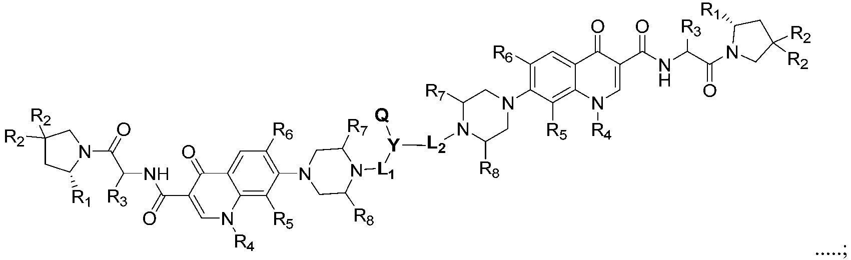
wherein, R₁ is selected from cyano;
R₂ is selected from hydrogen, fluorine or chlorine;
R₃ is selected from hydrogen or methyl;
R₄ is selected from hydrogen, methyl, ethyl, propyl, cyclopropyl or cyclobutyl;
R₅ and R₆ are independently selected from hydrogen, methyl, fluorine or chlorine;
R₇ and R₈ are independently selected from hydrogen or methyl;
L₁ and L₂ are independently selected from any one of the following structures: 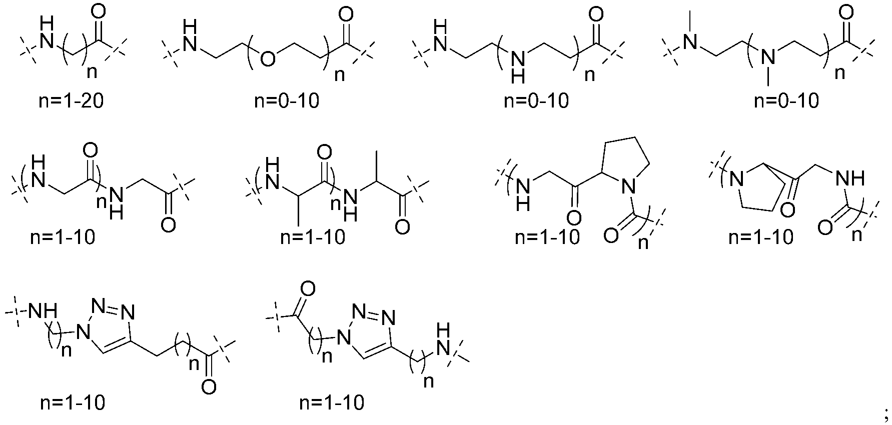
Y is selected from any one of: 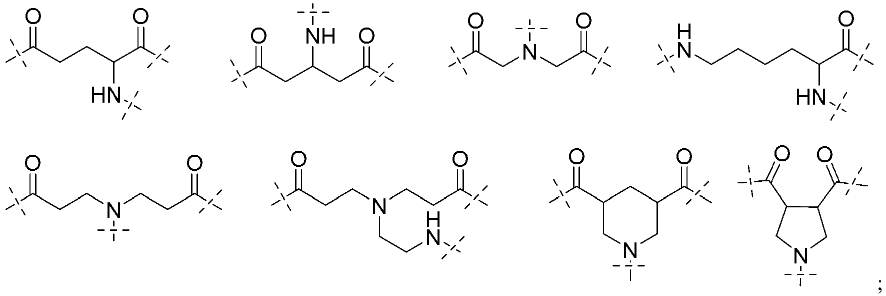
Q is a hydrogen atom, or as a nuclide chelating group moiety, is selected from any one of:

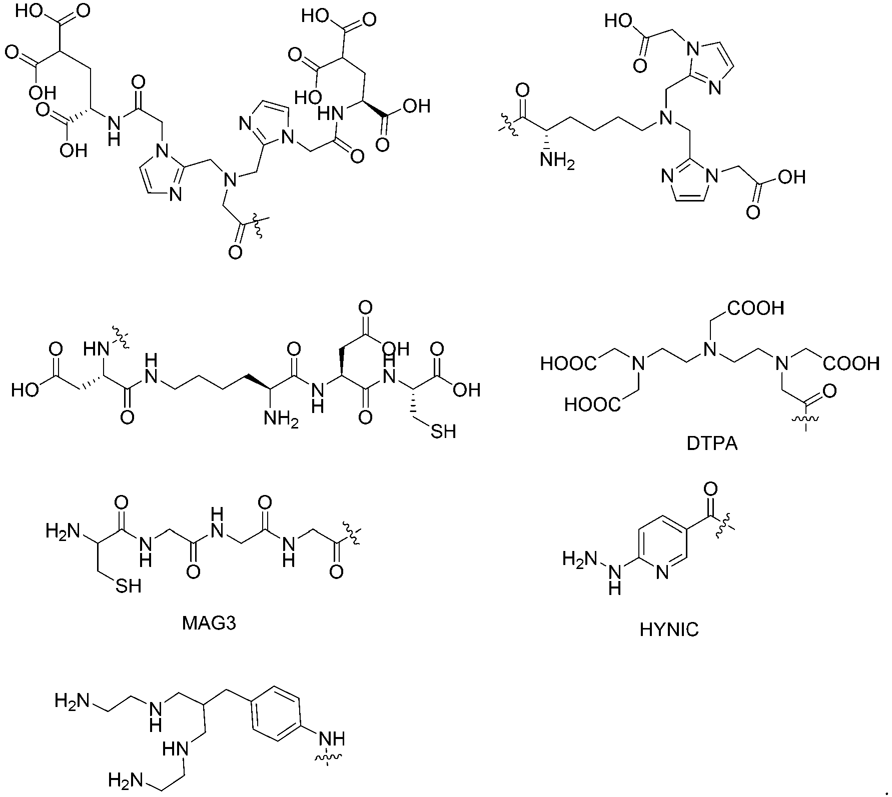

2. A FAP-targeting dimer compound or a pharmaceutically acceptable salt thereof, wherein the dimer compound is selected from any one of the following compounds:

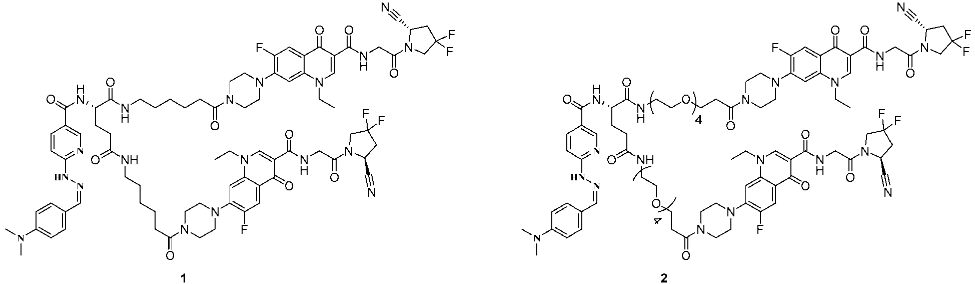

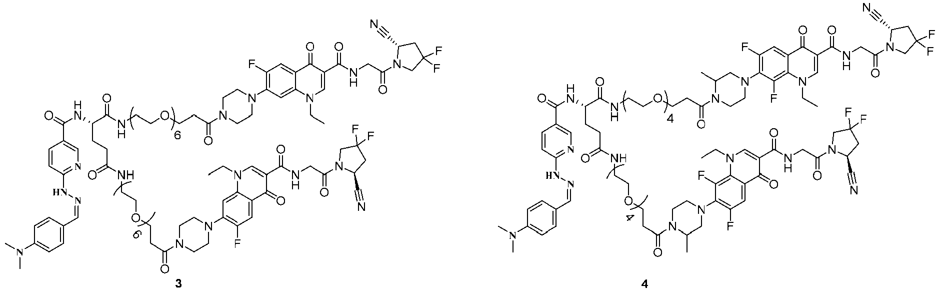

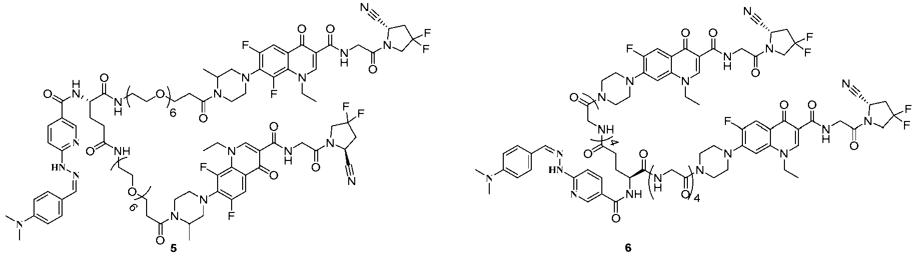

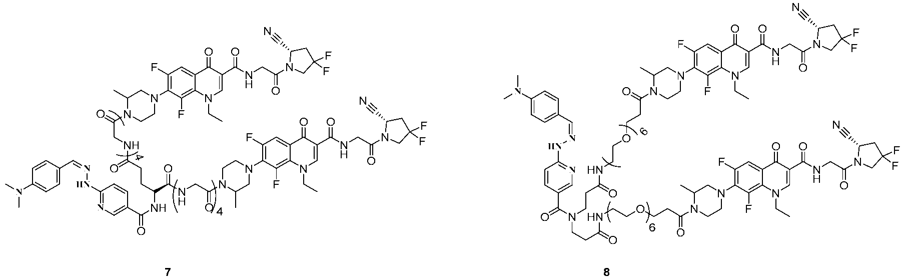

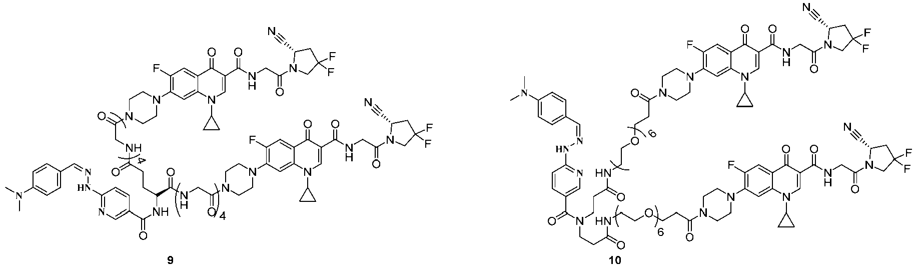

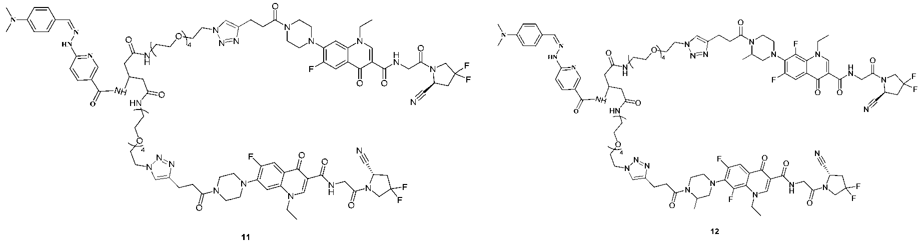

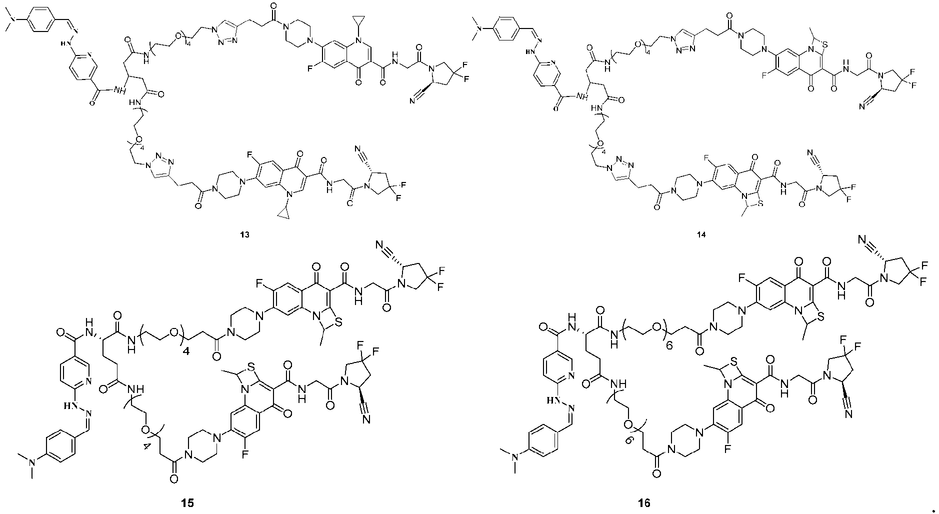

3. The FAP-targeting dimer compound or the pharmaceutically acceptable salt thereof according to claim 1 or 2, wherein the pharmaceutically acceptable salt is selected from hydrochlorides, sulfates, trifluoroacetates, fumarates, succinates, sulfonates, maleates, acetates, phosphates or citrates.

4. A radioactive nuclide technetium-99m labeled FAP-targeting compound, wherein the FAP-targeting dimer compound or the pharmaceutically acceptable salt thereof according to claim 1 or 2 reacts with a compound containing radioactive nuclide technetium-99m according to the existing wet labeling method or lyophilized labeling method, so as to prepare the radioactive nuclide technetium-99m labeled FAP-targeting compound.

5. A 99m-Tc labeled kit, comprising the FAP-targeting dimer compound or the pharmaceutically acceptable salt thereof according to any one of claims 1 to 3 or the radioactive nuclide technetium-99m labeled FAP-targeting compound according to claim 4, a ligand forming coordination with technetium-99m, a reducing agent, an additive and a stabilizer.

6. Use of the FAP-targeting dimer compound according to any one of claims 1 to 3 or the radioactive nuclide technetium-99m labeled FAP-targeting compound according to claim 4 or the kit according to claim 5 in preparation of drugs or reagents for treating and/or diagnosing diseases **characterized by** FAP positivity.

7. The use according to claim 6, wherein the diseases **characterized by** FAP positivity comprise tumors highly expressing FAP.
